# EUROPEAN PATENT APPLICATION

(11) **EP 0 976 824 A1**
(43) Date of publication of application: **02.02.2000**
(21) Application number: 98202336.8
(22) Date of filing: 10.07.1998
(51) Int. Cl.: C12N 15/12, C07K 14/47, C07K 16/18, C12Q 1/68

(54) **Gene and protein involved in liver regeneration**

(71) Applicant: Amsterdam Molecular Therapeutics, 3527 GA Utrecht (NL)
(72) Inventor: Chamuleau, Robert Antoine François Marie, 1412 JL Naarden (NL); Groenink, Martijn, 1019 RT Amsterdam (NL); Van der Vliet, Hendrik Niels, 1276 XZ Huizen (NL); Leegwater, Adam Cornelis Jozef, 1738 CR Waarland (NL)
(74) Representative: Van Someren, Petronella F. H. M.

(57) **Abstract**

Gene involved in regeneration processes of the liver and comprising a nucleotide sequence which is at least 70% homologous to the sequence of figure 1, or the complementary strand thereof, for use in the design of PCR probes for detecting nucleotide sequences in a source material, which nucleotide sequences represent genes corresponding with the gene sequence of figure 1; protein encoded by said gene for use in diagnosis of liver regeneration and/or liver cell proliferation; and antibodies directed against this protein, a PCR primer comprising at least part of said gene as a probe, and a single stranded nucleotide sequence being at least in part complementary to the messenger RNA transcribed from said gene as a probe, for use in a method for detecting the occurrence of liver cell proliferation in a subject.

SEQUENCE LISTING

(1) GENERAL INFORMATION:

(i) APPLICANT:
(A) NAME: Amsterdam Molecular Therapeutics
(B) STREET: Postbus 8323
(C) CITY: Utrecht
(E) COUNTRY: The Netherlands
(F) POSTAL CODE (ZIP): 3503 RH
(G) TELEPHONE: 020-5665861
(H) TELEFAX: 020-6916531

(ii) TITLE OF INVENTION: New gene and protein involved in
liver regeneration

(iii) NUMBER OF SEQUENCES: 21

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30
(EPO)

(v) CURRENT APPLICATION DATA:
APPLICATION NUMBER: EP 0 98202336.8

(2) INFORMATION FOR SEQ ID NO: 1:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid

(xi) SEQUENCE DESCRIPTION: SEQ ID NO: 1:

GCATCGTGGA AAGCATGGCT
20

(2) INFORMATION FOR SEQ ID NO: 2:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid

(xi) SEQUENCE DESCRIPTION: SEQ ID NO: 2:

GGGACCCTTG AGAGAGCCTG
20

(2) INFORMATION FOR SEQ ID NO: 3:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid

(xi) SEQUENCE DESCRIPTION: SEQ ID NO: 3:

CTTGAGGCAG CAGTTGAAAC
20

(2) INFORMATION FOR SEQ ID NO: 4:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid

(xi) SEQUENCE DESCRIPTION: SEQ ID NO: 4:

TCCACCCTTA TGCAGAACGC
20

(2) INFORMATION FOR SEQ ID NO: 5:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid

(xi) SEQUENCE DESCRIPTION: SEQ ID NO: 5:

AGTACCTTCA TCCGTGTCAG
20

(2) INFORMATION FOR SEQ ID NO: 6:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid

(xi) SEQUENCE DESCRIPTION: SEQ ID NO: 6:

CGCCTTCGCT CCAGAGTTGG
20

(2) INFORMATION FOR SEQ ID NO: 7:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid

(xi) SEQUENCE DESCRIPTION: SEQ ID NO: 7:

AGGGTGGAGG GTCCTGCATA
20

(2) INFORMATION FOR SEQ ID NO: 8:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid

(xi) SEQUENCE DESCRIPTION: SEQ ID NO: 8:

GCAAGCCAGT ACTTGACCGT
20

(2) INFORMATION FOR SEQ ID NO: 9:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid

(xi) SEQUENCE DESCRIPTION: SEQ ID NO: 9:

GTGGTCCTGC TGGGGGATCA
20

(2) INFORMATION FOR SEQ ID NO: 10:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid

(xi) SEQUENCE DESCRIPTION: SEQ ID NO: 10:

CAGGCTCTCT CAAGGGTCCC
20

(2) INFORMATION FOR SEQ ID NO: 11:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid

(xi) SEQUENCE DESCRIPTION: SEQ ID NO: 11:

CTACCTGCTC CATCAGCTCG
20

(2) INFORMATION FOR SEQ ID NO: 12:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid

(xi) SEQUENCE DESCRIPTION: SEQ ID NO: 12:

AGAGTTCTTT GACTCGGTCC
20

(2) INFORMATION FOR SEQ ID NO: 13:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid

(xi) SEQUENCE DESCRIPTION: SEQ ID NO: 13:

GAGCTCATCT CGCAGCTGAT
20

(2) INFORMATION FOR SEQ ID NO: 14:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid

(xi) SEQUENCE DESCRIPTION: SEQ ID NO: 14:

CTGTGGCTAG GCGGGGGTGG
20

(2) INFORMATION FOR SEQ ID NO: 15:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid

(xi) SEQUENCE DESCRIPTION: SEQ ID NO: 15:

CTGCCTATTA GGCCATGCTG
20

(2) INFORMATION FOR SEQ ID NO: 16:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid

(xi) SEQUENCE DESCRIPTION: SEQ ID NO: 16:

AGTCAGTCTC CCCCGCACAC
20

(2) INFORMATION FOR SEQ ID NO: 17:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid

(xi) SEQUENCE DESCRIPTION: SEQ ID NO: 17:

TGGCAGGGAT GTACACACTC
20

(2) INFORMATION FOR SEQ ID NO: 18:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid

(xi) SEQUENCE DESCRIPTION: SEQ ID NO: 18:

TTTCCATCAT GAGCGTCTAT
20

(2) INFORMATION FOR SEQ ID NO: 19:

(i) SEQUENCE CHARACTERISTICS:
(A) LENGTH: 1282 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO: 19:

GCATCGTGGA AAGCATGGCT GCCGTCATCA CCTGGGCACT CGCCCTCCTC TCAGTG-
TTTG 60

CAACTGTACA GGCGAGGAAG AGCTTCTGGG AGTACTTCGG CCAGAACAGC CAGGGC-
AAAG 120

GCATGATGGG CCAGCAGCAG AAGCTGGCAC AGGAGAGCCT GAAAGGTAGC TTGGAG-
CAAG 180

ACCTCTACAA TATGAACAAT TTCCTAGAAA AGCTGGGACC CTTGAGAGAG CCTGGG-
AAGG 240

AGCCTCCTCG GCTGGCACAG GATCCAGAAG GCATTCGGAA GCAGTTGCAG CAAG-
AGCTGG 300

AGGAAGTGAG CACACGCCTG GAGCCCTACA TGGCTGCAAA GCACCAGCAG GTCGGC-
TGGA 360

ACCTGGAGGG CTTGAGGCAG CAGTTGAAAC CCTACACGGT CGAGCTGATG GAG-
CAGGTAG 420

GCCTGAGCGT GCAGGATCTG CAAGAACAGC TGCGCATGGT GGGAAAAGGC AC-
CAAGGCCC 480

AGCTCCTGGG GGGCGTGGAT GAGGCGATGA GCCTGCTGCA GGATATGCAA AGTCGA-
GTGC 540

TGCACCATAC GGACCGAGTC AAAGAACTCT TCCACCCTTA TGCAGAACGC TTGGTG-
ACTG 600

GAATTGGGCA CCATGTGCAG GAGCTGCACC GGAGTGTTGC TCCTCACGCA GTTGCC-
AGCC 660

CCGCGAGACT CAGTCGCTGC GTGCAGACCC TGTCCCACAA ACTCACACGT AAGGCG-
AAGG 720

ACTTGCACAC CAGCATCCAA CGCAACCTGG ATCAGCTGCG AGATGAGCTC AG-
TACCTTCA 780

TCCGTGTCAG CACAGACGGG GCAGACAACA GAGACTCCCT GGACCCTCAA GCTCTC-
TCTG 840

ACGAGGTCCG CCAGAGACTC CAGGCTTTTC GACATGACAC CTACCTGCAG ATCGCT-
GCAT 900

TCACTCAGGC CATTGACCAG GAGACCGAGG AAATCCAGCA CCAGCTGGCA CCACCC-
CCGC 960

CTAGCCACAG CGCCTTCGCT CCAGAGTTGG GACACTCAGA CAGTAATAAG GCCCTG-
AGCA 1020

GACTGCAGAG CCGGCTGGAC GACCTCTGGG AAGATATTGC CTATGGCCTT CATGAC-
CAGG 1080

GCCATAGTCA GAATAACCCT GAGGGTCACT CAGGTTAACT CTGCAGCTCG TTGTCT-
GGAC 1140

CCTGAGCCTT CAGCATGGCC TAATAGGCAG AGGGTGGAGG GTCCTGCATA CTATTG-
GCGA 1200

GGCCACCAAA GGTGCTGCTG CCCCAACCTG TCTGGCCTCC TCAACTCCCC CACT-
CAGGTG 1260

CATTACACTC AGTAGGTTTG GC
1282

(2) INFORMATION FOR SEQ ID NO: 20:

(i) SEQUENCE CHARACTERISTICS:
(A) LENGTH: 1834 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO: 20:

GCATCGTGGA AAGCATGGCT GCCGTCATCA CCTGGGCACT CGCCCTCCTC TCAGTG-
TTTG 60

CAACTGTACA GGCGAGGAAG AGCTTCTGGG AGTACTTCGG CCAGAACAGC CAGGGC-
AAAG 120

GCATGATGGG CCAGCAGCAG AAGCTGGCAC AGGAGAGCCT GAAAGGTAGC TTGGAG-
CAAG 180

ACCTCTACAA TATGAACAAT TTCCTAGAAA AGCTGGGACC CTTGAGAGAG CCTGGG-
AAGG 240

AGCCTCCTCG GCTGGCACAG GATCCAGAAG GCATTCGGAA GCAGTTGCAG CAAG-
AGCTGG 300

AGGAAGTGAG CACACGCCTG GAGCCCTACA TGGCTGCAAA GCACCAGCAG GTCGGC-
TGGA 360

ACCTGGAGGG CTTGAGGCAG CAGTTGAAAC CCTACACGGT CGAGCTGATG GAG-
CAGGTAG 420

GCCTGAGCGT GCAGGATCTG CAAGAACAGC TGCGCATGGT GGGAAAAGGC AC-
CAAGGCCC 480

AGCTCCTGGG GGGCGTGGAT GAGGCGATGA GCCTGCTGCA GGATATGCAA AGTCGA-
GTGC 540

TGCACCATAC GGACCGAGTC AAAGAACTCT TCCACCCTTA TGCAGAACGC TTGGTG-
ACTG 600

GAATTGGGCA CCATGTGCAG GAGCTGCACC GGAGTGTTGC TCCTCACGCA GTTGCC-
AGCC 660

CCGCGAGACT CAGTCGCTGC GTGCAGACCC TGTCCCACAA ACTCACACGT AAGGCG-
AAGG 720

ACTTGCACAC CAGCATCCAA CGCAACCTGG ATCAGCTGCG AGATGAGCTC AG-
TACCTTCA 780

TCCGTGTCAG CACAGACGGG GCAGACAACA GAGACTCCCT GGACCCTCAA GCTCTC-
TCTG 840

ACGAGGTCCG CCAGAGACTC CAGGCTTTTC GACATGACAC CTACCTGCAG ATCGCT-
GCAT 900

TCACTCAGGC CATTGACCAG GAGACCGAGG AAATCCAGCA CCAGCTGGCA CCACCC-
CCGC 960

CTAGCCACAG CGCCTTCGCT CCAGAGTTGG GACACTCAGA CAGTAATAAG GCCCTG-
AGCA 1020

GACTGCAGAG CCGGCTGGAC GACCTCTGGG AAGATATTGC CTATGGCCTT CATGAC-
CAGG 1080

GCCATAGTCA GAATAACCCT GAGGGTCACT CAGGTTAACT CTGCAGCTCG TTGTCT-

GGAC    1140

CCTGAGCCTT CAGCATGGCC TAATAGGCAG AGGGTGGAGG GTCCTGCATA CTATTG-
GCGA    1200

GGCCACCAAA GGTGCTGCTG CCCCAACCTG TCTGGCCTCC TCAACTCCCC CACT-
CAGGTG    1260

CATTACACTC AGTAGGTTTG GCAAACACAG CTTCCGGTGC TCATTTGGGA TCCTAA-
GGAG    1320

CAAGAGTGGG GTGAAGGGAG TGGGGAGATG GTGTGCGGGG GAGACTGACT GCAAGC-
CAGT    1380

ACTTGACCGT TGCTAGAAAC CTGTGTCACT ACAACCTGGA GCCCGGCTCC TAT-
TACTTCA    1440

TGCCTGATGG TCGCTGTTAT AGTCGGTCTA CAGAGGGGAA CTCCTGTCTC CCCAGG-
GTTG    1500

TCATGACAGC CTTTGTTGGA AGAGAGCAGG AGAACATGAC ACGTATGATG GAGTGT-
GTAC    1560

ATCCCTGCCA GTGGTCCTGC TGGGGGAATC AGTGATGGGA TAAATGTGTG CATCCC-
TGCA    1620

GTGGTCCTGC TGGGGGATCA GTGATGGGAT GGGGCAGAGC CCCTATTTCC TTAGA-
GAACT    1680

CTAACCCAAA TAAGGAACTG AGCCCTCTGC AGTGAGGGCT TCTGAAAACC CTGTA-
CATAG    1740

CAAACTGTGT GCCCTCTTCA TCATGCAGTC CCCACCTCCT GATTCTCGGG ATGGAA-
CTGA    1800

CTTTTGGTTG GAATGAAATA GACGCTCATG ATGG
    1834

(2) INFORMATION FOR SEQ ID NO: 21:

(i) SEQUENCE CHARACTERISTICS:
(A) LENGTH: 367 amino acids
(B) TYPE: amino acid
(C) STRANDEDNESS:
(D) TOPOLOGY: unknown

(ii) MOLECULE TYPE: protein

(xi) SEQUENCE DESCRIPTION: SEQ ID NO: 21:

Met Ala Ala Val Ile Thr Trp Ala Leu Ala Leu Leu Ser Val
Phe Ala
1               5                   10
15

Thr Val Gln Ala Arg Lys Ser Phe Trp Glu Tyr Phe Gly Gln
Asn Ser
            20                  25                  30

Gln Gly Lys Gly Met Met Gly Gln Gln Gln Lys Leu Ala Gln
Glu Ser
        35                  40                  45

Leu Lys Gly Ser Leu Glu Gln Asp Leu Tyr Asn Met Asn Asn
Phe Leu
    50                  55                  60

Glu Lys Leu Gly Pro Leu Arg Glu Pro Gly Lys Glu Pro Pro
Arg Leu
65                  70                  75
80

Ala Gln Asp Pro Glu Gly Ile Arg Lys Gln Leu Gln Gln Glu
Leu Glu
                85                  90
95

Glu Val Ser Thr Arg Leu Glu Pro Tyr Met Ala Ala Lys His
Gln Gln
            100                 105                 110

Val Gly Trp Asn Leu Glu Gly Leu Arg Gln Gln Leu Lys Pro
Tyr Thr
        115                 120                 125

Val Glu Leu Met Glu Gln Val Gly Leu Ser Val Gln Asp Leu
Gln Glu
    130                 135                 140

Gln Leu Arg Met Val Gly Lys Gly Thr Lys Ala Gln Leu Leu
Gly Gly
145                 150                 155
160

Val Asp Glu Ala Met Ser Leu Leu Gln Asp Met Gln Ser Arg
Val Leu
                165                 170
175

His His Thr Asp Arg Val Lys Glu Leu Phe His Pro Tyr Ala
Glu Arg
180 185 190

Leu Val Thr Gly Ile Gly His His Val Gln Glu Leu His Arg
Ser Val
195 200 205

Ala Pro His Ala Val Ala Ser Pro Ala Arg Leu Ser Arg Cys
Val Gln
210 215 220

Thr Leu Ser His Lys Leu Thr Arg Lys Ala Lys Asp Leu His
Thr Ser
225 230 235
240

Ile Gln Arg Asn Leu Asp Gln Leu Arg Asp Glu Leu Ser Thr
Phe Ile
245 250
255

Arg Val Ser Thr Asp Gly Ala Asp Asn Arg Asp Ser Leu Asp
Pro Gln
260 265 270

Ala Leu Ser Asp Glu Val Arg Gln Arg Leu Gln Ala Phe Arg
His Asp
275 280 285

Thr Tyr Leu Gln Ile Ala Ala Phe Thr Gln Ala Ile Asp Gln
Glu Thr
290 295 300

Glu Glu Ile Gln His Gln Leu Ala Pro Pro Pro Pro Ser His
Ser Ala
305 310 315
320

Phe Ala Pro Glu Leu Gly His Ser Asp Ser Asn Lys Ala Leu
Ser Arg
325 330
335

Leu Gln Ser Arg Leu Asp Asp Leu Trp Glu Asp Ile Ala Tyr
Gly Leu
340 345 350

His Asp Gln Gly His Ser Gln Asn Asn Pro Glu Gly His Ser
Gly
355 360 365

## Description

The present invention relates to the detection of a novel gene and protein involved in liver cell proliferation. The gene and protein and related molecules, such as nucleotide probes derived from the gene and antibodies directed to the protein form also part of the invention. The gene will be identified herein as RAP3 gene. The corresponding protein is called rap3 protein.

The adult liver has the capacity to regenerate after damage or partial resection. This process may allow for recovery from hepatic injuries caused by viruses, toxins, ischemia, surgery, and auxiliary liver transplantation. Liver regeneration has been studied extensively in the rat after a 70% partial hepatectomy. During the first four hours following partial hepatectomy there is a rapid, transient transcriptional activation of genes involved in the immediate early response. After induction of these immediate early genes during the transition from the quiescent state of the liver (G₀) to the growth phase (G₁), a delayed early gene activation is initiated which peaks during the transition of the G₁ to the DNA synthesis phase (S phase).

In the research that led to the present invention novel genes involved in the delayed early response were identified by analyzing gene expression in rat liver at six hours after 70% partial hepatectomy. Upregulated genes were selected by cDNA subtractive hybridization. Upregulation was quantified by Northern blotting and the truly upregulated genes were characterized by sequence analysis.

Twelve genes were found to be upregulated at different degrees (1.5 to 10.4 fold) six hours after partial hepatectomy. Sequence analysis revealed that eight of the upregulated genes have previously been reported to be associated with liver regeneration or cell proliferation in general, one has previously been assigned an unrelated function and three have no sequence similarity to known genes.

The various upregulated genes showed two distinct gene expression patterns during a 30 hour period after partial hepatectomy. The first pattern has two peaks coincident with the G₁ phases of two consecutive hepatic cell cycles. The second one shows a narrow peak at six hours after which the gene is downregulated. The novel gene which was most upregulated (3.3 fold), showed the latter gene expression pattern.

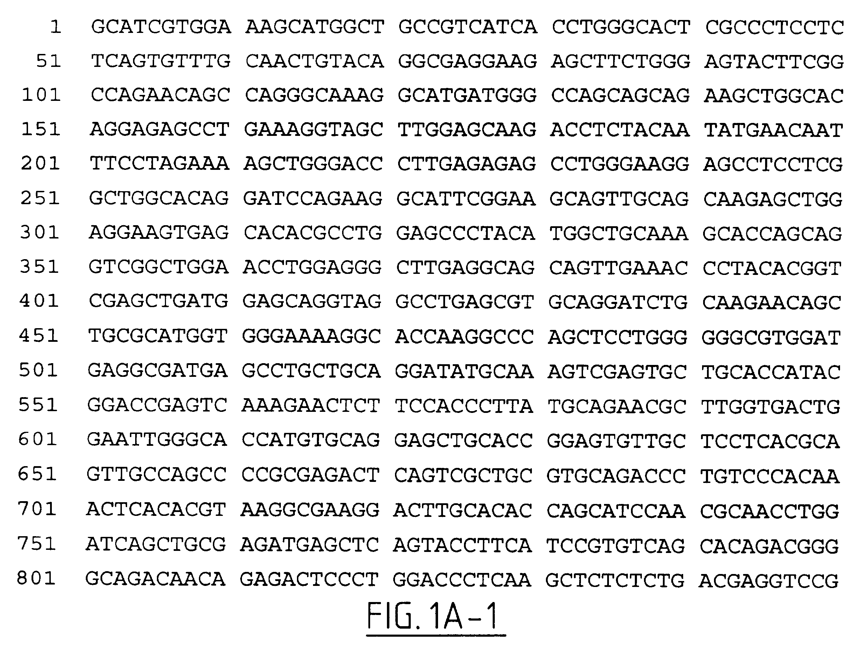

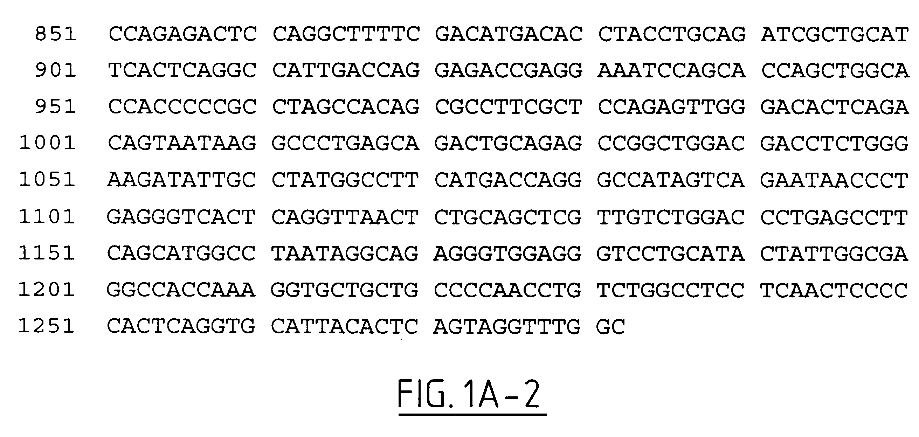

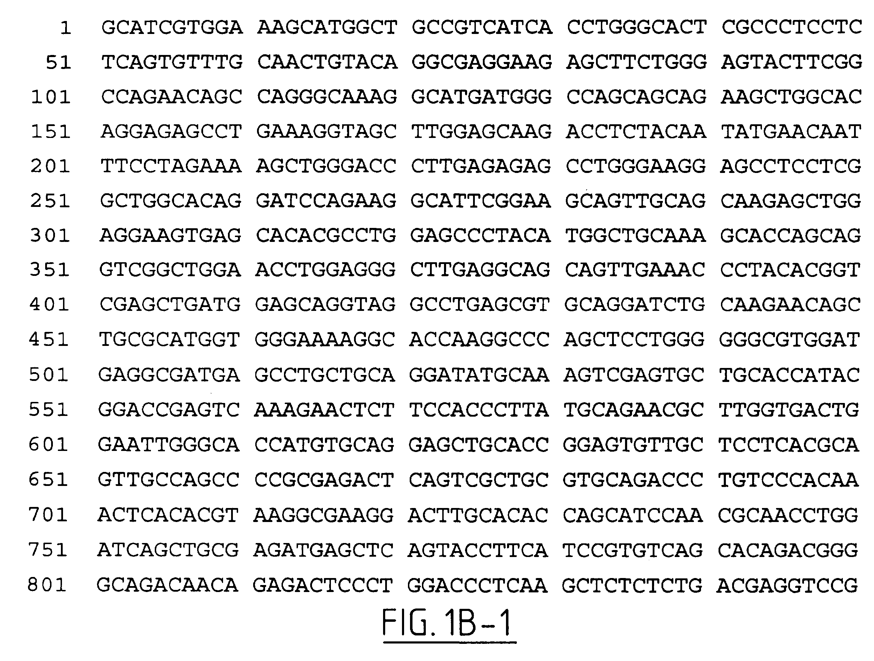

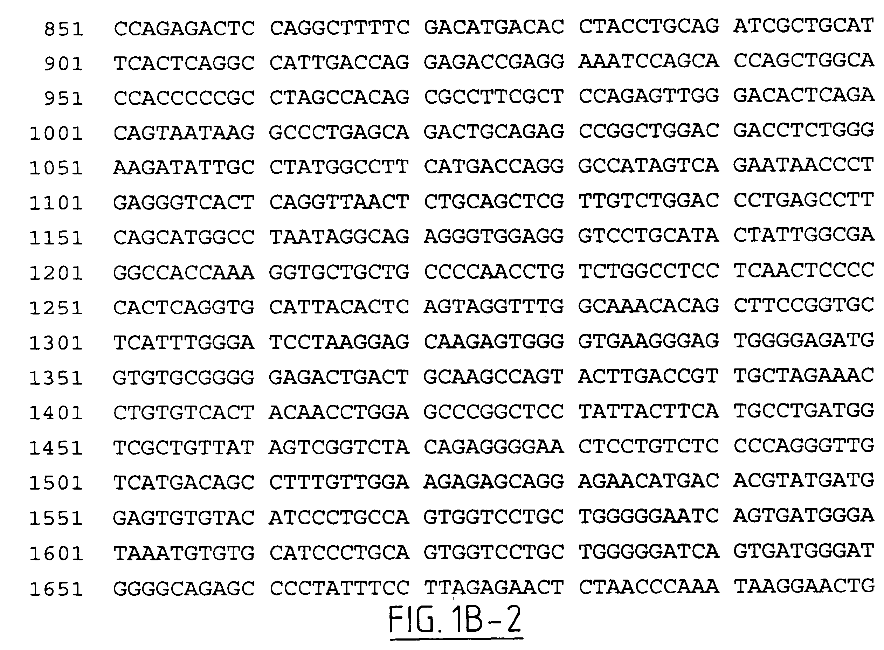

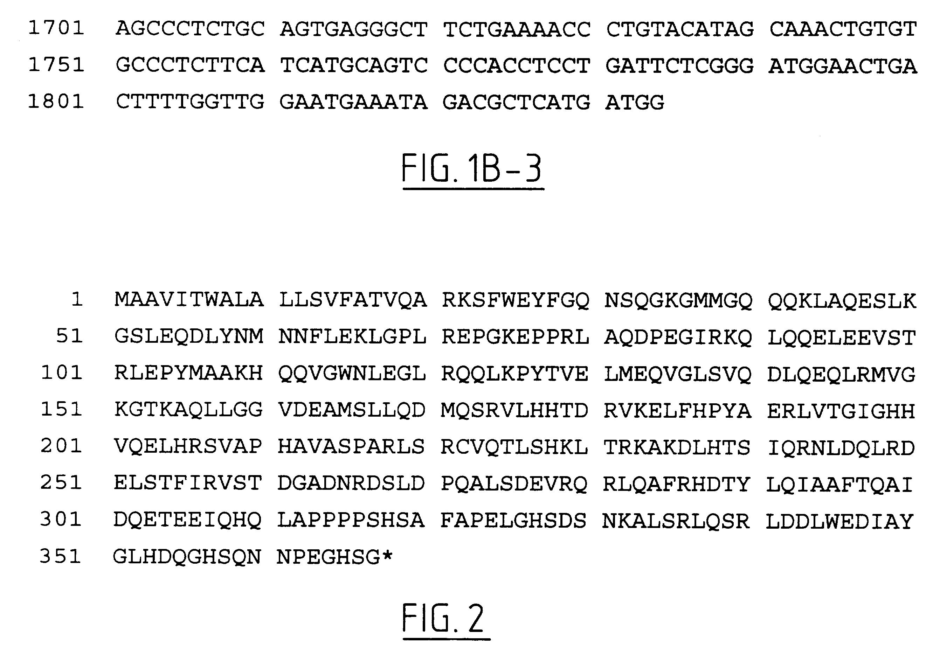

The full length cDNA of this gene was isolated from a rat liver cDNA library. Sequence analysis showed two full length cDNA's of 1282 and 1834 bp, respectively, encoding a novel protein of 367 amino acid residues. Figures 1A and 1B show the nucleotide sequence of the cDNA's. Figure 2 shows the derived amino acid sequence.

On the basis of this finding it became possible to design probes, primers and reagents for use in diagnosis. Furthermore, based on the general 70% homology between the rat and human genome the corresponding human gene can be isolated.

Probes and primers are generally based on the nucleotide sequence of the gene. Hybridization probes can comprise the whole or a large part of the coding or complementary strand of the sequence. PCR primers are typically smaller and encompass about between 10 and 50, preferably between 15 and 30, more preferably about 20 nucleotides.

The nucleotide sequences of some suitable PCR primers are given in the following table.

**Table I**

| **primer name** | **nucleotide sequence** |
|---|---|
| F1RAP | 5' GCA TCG TGG AAA GCA TGG CT 3' |
| F215RAP | 5' GGG ACC CTT GAG AGA GCC TG 3' |
| F371RAP | 5' CTT GAG GCA GCA GTT GAA AC 3' |
| F571RAP | 5' TCC ACC CTT ATG CAG AAC GC 3' |
| F771RAP | 5' AGT ACC TTC ATC CGT GTC AG 3' |
| F971RAP | 5' CGC CTT CGC TCC AGA GTT GG 3' |
| F1171RAP | 5' AGG GTG GAG GGT CCT GCA TA 3' |
| F1371RAP | 5' GCA AGC CAG TAC TTG ACC GT 3' |
| F1621RAP | 5' GTG GTC CTG CTG GGG GAT CA 3' |
| R234RAP | 5' CAG GCT CTC TCA AGG GTC CC 3' |
| R420RAP | 5' CTA CCT GCT CCA TCA GCT CG 3' |
| R570RAP | 5' AGA GTT CTT TGA CTC GGT CC 3' |
| R770RAP | 5' GAG CTC ATC TCG CAG CTG AT 3' |
| R970RAP | 5' CTG TGG CTA GGC GGG GGT GG 3' |
| R1170RAP | 5' CTG CCT ATT AGG CCA TGC TG 3' |
| R1370RAP | 5' AGT CAG TCT CCC CCG CAC AC 3' |
| R1570RAP | 5' TGG CAG GGA TGT ACA CAC TC 3' |
| R1837RAP | 5' TTT CCA TCA TGA GCG TCT AT 3' |

The hybridization probes can be labeled with a detectable label, such as a radioactive or biotin label.

Diagnosis of expression of the gene can be performed by means of a Northern blot. Total RNA or mRNA of a sample is separated on an agarose gel. The separation pattern is transferred to a nylon or nitrocellulose filter. An increase or decrease in the expression level is subsequently detected by hybridization with the above described hybridization probe. Typically a reference sample is included for comparison.

In case the protein is the basic macromolecule for diagnosis polyclonal or monoclonal antibodies are used for detection. The skilled person is very well capable of preparing such antibodies based on his common knowledge. Antibodies against the protein are part of the present invention.

Samples to be diagnosed can be a liver biopsy, plasma or serum. The latter can be used because the protein is secreted in the blood stream.

With the above described diagnostic methods an increase or decrease in the expression of the gene of the invention can be detected. The information that can thus be obtained is useful for establishing the efficacy of therapeutic agents stimulating liver regeneration and for patients who underwent an (auxiliary) liver transplantation and for monitoring patients treated with a bioartificial liver.

The invention is further illustrated in the following examples, which are in no way intended to be limiting to the invention. In the examples reference is made to the following figures:
Figure 1A is the nucleotide sequence of the 1282 bp cDNA.
Figure 1B is the nucleotide sequence of the 1834 bp cDNA.
Figure 2 shows the deduced amino acid sequence of the rap3 protein.
Figure 3 shows a polyacrylamide gel of liver cDNA fragments before and after subtraction. 26 cDNA fragments were found to be enriched after subtraction. Some of these are indicated by arrows. Lane 1 shows liver cDNA fragments of 6 hours 70% partial hepatectomy before subtraction. Lane 2 shows cDNA fragments of 6 hours 70% partial hepatectomy after subtraction.
Figure 4 shows the results of the Northern blot analysis of the temporal expression of RAP3 up to 30 hours after 70% partial hepatectomy. Panel A represents the Northern blot mRNA expression patterns at 3, 6, 12, 18, 24 and 30 hours after the 70% hepatectomy (hpx) and laparotomy (sham). Panel B represents the quantified hybridization signals indicated in PhosphorImager arbitrary units obtained at 6, 12, 18, 24 and 30 hours after the 70% hepatectomy and laparotomy.
   The novel gene RAP3 is mostly upregulated 6 hours after partial hepatectomy after which it becomes downregulated.
Figure 5 shows a rat tissue Northern blot hybridized with a RAP3 cDNA probe. The RAP3 gene is specifically expressed in the liver.

### EXAMPLES

### EXAMPLE 1

### Isolation of RAP3 gene associated with liver regeneration

### 1. Introduction

Recovery from Hepatic injuries caused by viruses, toxins, ischemia, surgery and auxiliary liver transplantation can be achieved by regeneration of the liver. The regeneration process has been studied extensively in the rat after a 70% partial hepatectomy.

During the first four hours following partial hepatectomy there is a rapid, transient transcriptional response. After this induction during the transition from the quiescent state of the liver (G₀) to the growth phase (G₁), a delayed early gene activation is initiated, which peaks during the transition of the G₁ to the DNA synthesis phase (S phase).

This example demonstrates the isolation and identification of genes which are upregulated in the regenerating liver 6 hours after 70% partial hepatectomy.

### 2. Methods

### 2.1 Rat liver tissue preparation

Experiments were carried out in compliance with the guidelines on the care and use of laboratory animals of the University of Amsterdam. Regenerating liver was obtained from male Wistar rats (200-225 g). Rats were anesthetized with ether and subjected to midventral laparotomy. Subsequently, the left lateral and the median liver lobes were removed (70% partial hepatectomy) (G.M. Higgins and R.M. Anderson, Arch. Pathol. **12**, 186 (1931)). For sham-operated animals, the liver was exposed by a midventral laparotomy.

The rats were allowed to recover from anesthesia. At 3, 6, 12, 18, 24, and 30 hours, respectively, after the 70% partial hepatectomy and sham surgery the animals were killed and the remaining liver was immediately harvested.

### 2.2 RNA isolation

Total liver RNA was isolated from liver tissue using the Trizol reagent kit (Life Technologies). Liver poly A⁺ RNA was isolated from total liver RNA using oligo(dT)-cellulose (Boehringer Mannheim GmbH) affinity chromatography as described previously (Maniatis, T., Fritsch, E.F., and Sambrook, J. (1982) Molecular cloning: A laboratory Manual, Cold Spring Harbor, NY). To obtain highly pure poly A⁺ RNA populations the oligo-(dT)-cellulose step was performed twice. The integrity of the poly A⁺ RNA populations was determined on Northern blot by hybridization with glutathione-S transferase (data not shown).

### 2.3 PCR-select cDNA subtraction

The PCR-select cDNA subtraction kit (Clontech) was used to selectively amplify delayed early genes differentially expressed during liver regeneration. This method subtracts sequences common to both cDNA populations by suppressing undesirable PCR amplification, rather than by physically separating single stranded and double-stranded DNA. The 6 hours 70% partial hepatectomy liver poly A⁺ population, containing the differentially expressed mRNA's, was compared with the 6 hours laparotomy liver mRNA population. Delayed-early genes start to appear 3 to 4 hours after the 70% partial hepatectomy. By using a laparotomy liver mRNA population rather than a normal liver mRNA population, the two populations were equalized for acute phase mRNA's, which are induced by the operation itself.

The PCR-select cDNA subtraction was performed according to the manufacturer's protocol with the following modifications. After two hybridizations, a nested PCR was used to selectively amplify the differentially expressed sequences. The second, nested PCR was performed in the presence of 0.5 µM [α- ³³P]dATP (1200 Ci/mmol, final volume 25 µl). Subsequently, the amplified and differentially expressed cDNA fragments were visualized on a denaturing 4% polyacrylamide DNA sequencing gel. An X-ray film (Biomax, Kodak) was exposed overnight to the unfixed, dried gel.

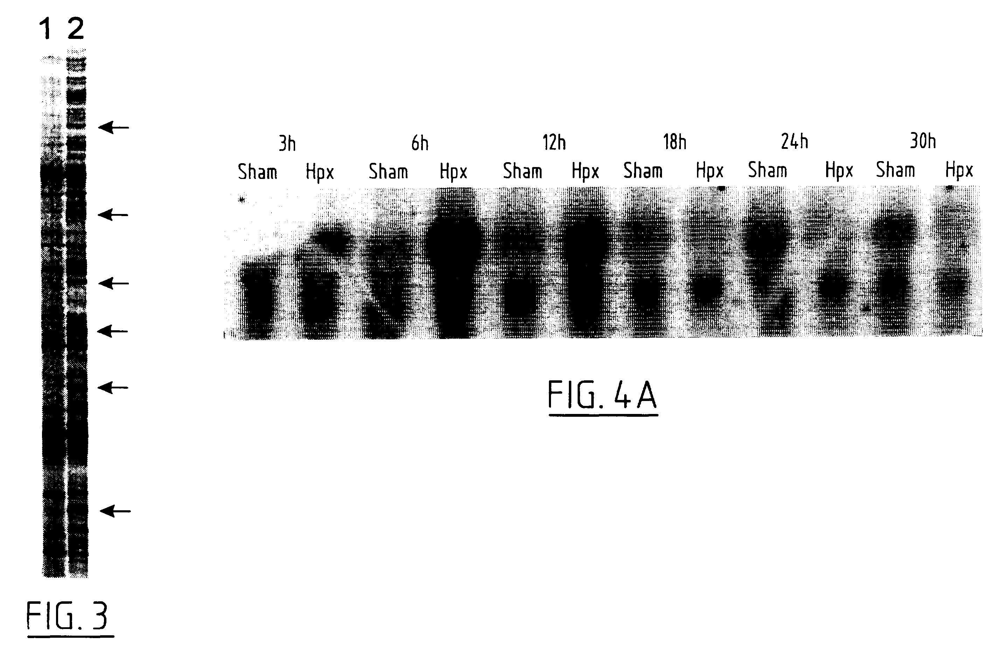

Figure 3 shows the results of the subtraction. Before subtraction (lane 1), the majority of the cDNA's were poorly identifiable, indicating the presence of many cDNA fragments of different molecular size. After subtraction (lane 2), 26 distinct cDNA fragments were observed as bands that were not apparent before subtraction.

### 2.4 Isolation and identification of visualized cDNA fragments

The 26 cDNA fragments that became visible after PCR-select cDNA subtraction were excised from the dried polyacrylamide gel and heated to 100°C for 5 minutes. Subsequently, 25 µl of the aqueous cDNA extract was used to amplify the cDNA by PCR with the nested primers used in the PCR-select cDNA subtraction. The PCR product was ligated into pCR II (Invitrogen), transformed into INVαF' competent cells, and plated out on agar plates containing ampicillin and X-Gal. Of each cloned PCR product, 6 white colonies were analyzed by PCR with T7 and SP6 primers for the presence of an insert.

Subsequently, plasmids containing an insert were purified using QIAprep (Qiagen) and the sequences of the inserts were determined using a dye terminator cycle sequencing system (Perkin Elmer) and a 377 DNA sequencer (ABI PRISM).

### 2.5 Northern blot analysis

To determine whether the expression of the genes found by the PCR-select subtractive hybridization is truly increased 6 hours after partial hepatectomy, Northern blot analysis was carried out using the purified cDNA fragments as probes. Poly A⁺ RNA samples (0.8 µg) of the liver 6 hours after the hepatectomy and sham operation were electrophoresed on a 0.22 M formaldehyde-1% agarose gel, and blotted onto a Hybond-N nylon membrane (Amersham) by capillary transfer overnight. For fixation of the poly A⁺ RNA the blots were baked in an oven at 80°C for 2 hours.

The inserts of the sequenced clones were amplified by PCR using the nested primers of the PCR-select cDNA subtraction method. Qiaquick-spin columns (Qiagen) were used to purify the PCR products. The purified PCR products were radioactively labelled according to the hexamer-random primed method following the manufacturer's protocol (Promega), purified on Qiaquick-spin columns (Qiagen), and hybridized with the blots. Prehybridization (2 hours, 42°C) and hybridization (overnight, 42°C) was performed in 5 x SSPE, 50% formamide, 5 x Denhardt, 0.5% SDS, and 0.1 mg/ml sheared heat-denatured herring sperm DNA.

Following hybridization the blots were washed with 2 x SSC and 0.1% SDS for 15 min at room temperature and 42°C, respectively. Subsequently, the solution was replaced with 1 x SSC and 0.1% SDS and the blots were washed for 15 min at room temperature and at 42°C, respectively. The amount of hybridization was analyzed and quantified using a PhosphorImager (Molecular Dynamics).

The fold induction of the mRNA levels observed in the 70% partially hepatectomized animals over the sham operated animals after the specific hybridization was adjusted for variability in RNA loading.

The genes which were upregulated 1.5 times or more 6 hours after 70% hepatectomy together with their identity are given in Table II. Beside these twelve genes, three genes are indicated which expression could not be detected on Northern blot. The expression of the novel RAP3 gene was found to be upregulated 3.3 fold.

**Table II**

| **GENES UPREGULATED 6 HOURS AFTER A 70%** | | |
|---|---|---|
| **Identity of gene** | **Function** | **Fold** |
| Fibronectin | Liver regeneration | 1.8 |
| An intracisternal-A | Liver regeneration | 1.8 |
| γ-Actin | Liver regeneration | 7 |
| Ribophorin I | Liver regeneration | 5.5, 1.7 & 2.3 |
| α₂-Macroglobulin | Hepatocyte proliferation in vitro | 5.4 |
| Ribosomal Protein S5 | Cell cycle | 3.7 & 1.9 |
| Ribosomal Protein L13 | Cell cycle | 2 |
| Amyloid A Protein | Growth factor | 10.4 |
| Entactin | | N.D. |
| TCP-1-Containing Chaperonin related gene | | 1.5 |
| 31 kDa Putative Serine/Threonine protein kinase | | N.D. |
| Novel RAP1 | Unknown | 1.5 |
| Novel RAP2 | Unknown | 1.6 |
| Novel RAP3 | Unknown | 3.3 |
| Novel RAP4 | Unknown | N.D. |

| | | |
|---|---|---|
| * N.D. = not detectable on Northern blot | | |

### EXAMPLE 2

### Isolation and characterization of the full length RAP3 cDNA

### Library screening and sequence analysis

A rat liver cDNA library was prepared from poly A⁺ RNA isolated from the rat liver 6 hours after 70% hepatectomy. To obtain full length cDNA, the Great Lengths cDNA Synthesis Kit (Clontech) was used following the manufacturer's protocol. The adaptor ligated full length cDNA inserts were cloned into the mammalian expression vector pCI at the EcoRI restriction site.

After transformation into DH10B electrocompetent cells (Gibco), the cDNA library was plated at a density of about 3,000 plaques per 150-mm-diameter petri dish. Colonies were lifted onto a Hybond-N nylon membrane (Amersham). The lift was hybridized with the ³²P-labeled RAP3 PCR fragment prepared according to the hexamer-random primed method following the manufacturer's protocol (Promega).

Following hybridization, the lift was washed and analyzed using a PhosphorImager (Molecular Dynamics). From the nine positive clones, the plasmid DNA was purified and the sequences of the inserts were determined using a dye terminator cycle sequencing system (Perkin Elmer) and a 377 DNA sequencer (ABI PRISM). The RAP3 cDNA was obtained by comparing the nine sequences with the sequence of the RAP3 PCR fragment. Two possible clones were detected and the start and end of the cDNA were confirmed by 5'- and 3'-RACE reactions carried out following the protocol of the Marathon cDNA Amplification kit (Clontech).

Based on the nucleotide sequence of the clones, PCR reactions were carried out with cDNA prepared from poly A⁺ RNA of the rat liver 6 hours after 70% hepatectomy. The PCR products comprised the whole RAP3 cDNA, of which the nucleotide sequence was determined by bidirectionally sequencing the PCR products using 20 bp primers based on the already known nucleotide sequence data of the RAP3 cDNA.

Two RAP3 cDNA molecules were detected of 1282 and 1834 bp respectively. The latter showed the same nucleotide sequence as the first, but contained an additional 552 bp nucleotide part at the 3' side.

The nucleotide sequence of the 1282 bp RAP3 cDNA is as shown in Figure 1A.

The nucleotide sequence of the 1834 bp RAP3 cDNA is shown in Figure 1B.

Using GCG DNA software the nucleotide sequences were translated into the amino acid sequence. By analyzing the six reading frames, the largest possible protein was chosen as the RAP3 protein. Its amino acid sequence, starting with a methionine residue and ending at a stop codon, was the most likely one to form a protein in comparison with the other smaller possible proteins. Both RAP3 cDNA molecules encode the same RAP3 protein.

The amino acid sequence of RAP3 protein as deduced from the nucleotide sequence is shown in Figure 2.

### EXAMPLE 3

### Temporal expression between 3 and 30 hours after 70% partial hepatectomy

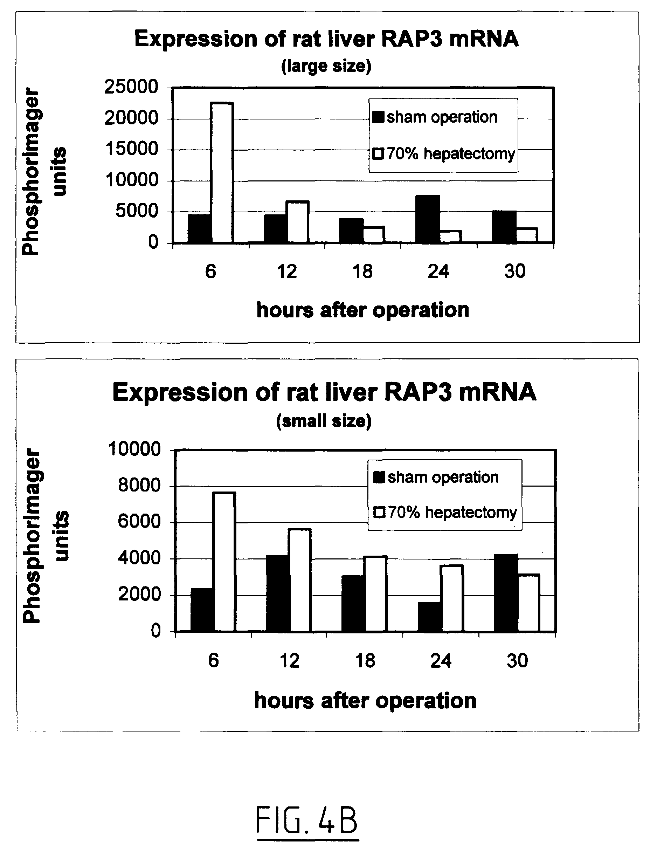

To define the temporal expression of the RAP3 gene, mRNA levels at 3, 6, 12, 18, 24, and 30 hours after the 70% partial hepatectomy and laparotomy were analyzed by the Northern blot procedure as described in example 1. Total RNA samples (20 µg) of the rat liver isolated at the various time points were electrophoresed rather than poly A⁺ RNA. The Northern blot was hybridized with a radioactively labeled probe comprising basepairs 370 to 1834 of the large RAP3 cDNA. The result of the Northern blot and the quantified expression pattern are given in Figure 4. The expression pattern is presented as the hybridization signal in PhosphorImager arbitrary units obtained at 3, 6, 12, 18, 24, and 30 hours after the 70% partial hepatectomy and laparotomy.

Both RAP3 mRNA sizes are mostly upregulated 6 hours after partial hepatectomy after which they become downregulated.

The same procedure was carried out with probes of the other upregulated genes obtained by the PCR-select subtraction. Two distinct gene expression patterns during the 30 hour period after partial hepatectomy were found. The first pattern has two peaks coincident with the G₁ phases of two consecutive hepatic cycles. The second one shows a narrow peak at six hours after which the gene is downregulated, just like the expression pattern of the novel RAP3 gene.

### Determination of tissue specific expression

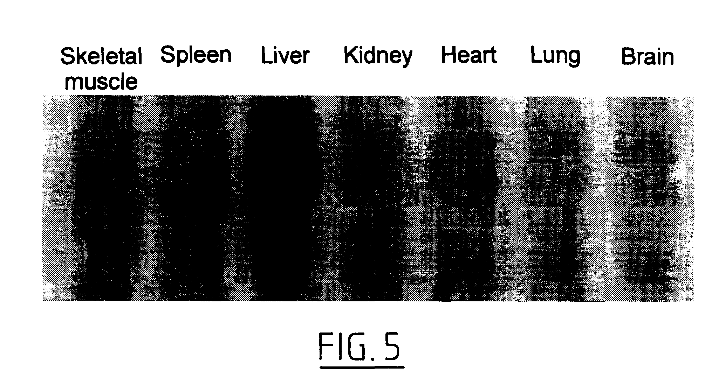

A Northern blot was prepared to determine expression of RAP3 mRNA in different tissues. The various tissues (skeletal muscle, spleen, liver, kidney, heart, lung and brain) were isolated from a female Wistar rat (175 g). The experiment was carried out in compliance with the guidelines on the care and use of laboratory animals of the University of Amsterdam. Total liver RNA was isolated from the tissues using the Trizol reagent kit (Life Technologies). A Northern blot was prepared from 20 µg total RNA samples and Northern blot analysis was carried out as described in example 1. A radioactively labeled probe comprising basepairs 370 to 1834 of the large RAP3 cDNA was used for the hybridization. The resulting Northern blot is given in Figure 5.

The RAP3 mRNA appeared to be clearly expressed in the liver and not at any detectable level in the other examined tissues. Because of this liver specificity and the 3.3 fold upregulation six hours after hepatectomy, the novel gene RAP3 was considered to be important in the process of liver regeneration.

### EXAMPLE 4

### Detection of changes of the amount of the RAP3 protein in the blood circulation

In order to detect changes in the amount of the RAP3 protein in the blood circulation a specific enzyme-linked immunosorbent assay (ELISA) is developed. Specific polyclonal and/or monoclonal antibodies are raised against the whole protein or a part of the protein. The protein, human or rat, is expressed in a prokaryotic or eukaryotic expression system or part of the protein is synthesized chemically. Monoclonal and polyclonal antibodies, raised in rabbits, are isolated by common techniques as described previously (Coligan, J.E., Kruisbeek, A.M., Margulies, D.M., Shevach, E.M., and Strober, W. (1994) Current Protocols in Immunology, John Wiley & Sons, Inc. Chicester, New York).

### EXAMPLE 5

### Isolation of the corresponding human gene

To obtain the human analogue of the RAP3 gene, a human liver cDNA library can be purchased. With this library a colony-hybridization screening is performed as described in example 2 for the detection of the rat RAP3 cDNA. Since human and rat genes have quite homologous nucleotide sequences, the rat RAP3 cDNA is used as a probe. In this way it is possible to isolate the human RAP3 gene from the cDNA library. To characterize the human RAP3 cDNA, it is sequenced as described in example 2. From the nucleotide sequence the amino acid sequence of the human RAP3 protein can be deduced.

## Claims

1. Gene involved in regeneration processes of the liver and comprising a nucleotide sequence which is at least 70% homologous to the sequence shown in Fig. 1 or the complementary strand thereof.

2. Gene as claimed in claim 1, **characterized in that** its cDNA has a nucleotide sequence which is at least 70% homologous to the nucleotide sequence as depicted in Fig. 1 or the complementary strand thereof.

3. Gene as claimed in claims 1 and 2 for use in the design of PCR probes for detecting nucleotide sequences in a source material, which nucleotide sequences represent genes corresponding with the gene sequence shown in Fig. 1.

4. Gene as claimed in claims 1 and 2 for use as a marker of liver proliferation.

5. Protein encoded by a gene as defined in claims 1 and 2 and comprising an amino acid sequence which is at least 70% homologous to the amino acid sequence given in Fig. 2.

6. Protein as claimed in claim 5 having the amino acid sequence as depicted in Fig. 2 or the complementary strand thereof.

7. Protein as claimed in claims 5 and 6 for use in diagnosis of liver regeneration and/or liver cell proliferation.

8. Antibodies directed against a protein as claimed in claims 5 and 6.

9. Antibodies as claimed in claim 7 for use in a method for detecting the occurrence of liver cell proliferation in a subject.

10. Antibodies as claimed in claim 8 or 9 which antibodies are monoclonal antibodies.

11. Antibodies as claimed in claim 8 or 9 which antibodies are polyclonal antibodies.

12. PCR primer, comprising at least part of the gene as claimed in claim 1.

13. PCR primer, comprising at least part of the nucleotide sequence as shown in Fig. 1 or its complementary strand.

14. PCR primer as claimed in claims 12 and 13, wherein the "at least part of the nucleotide sequence" encompasses 10 to 50, preferably 15 to 30, more preferably about 20 nucleotides.

15. PCR primer as claimed in claims 12 to 14 having the nucleotide sequence as depicted in Table I or the complementary strand thereof.

16. PCR primer as claimed in claims 12 to 15 for use as a probe in a method for detecting the occurrence of liver proliferation in a subject.

17. PCR primer as claimed in claims 12 to 15 for use in the detection of gene homologous to the gene as claimed in claims 1 to 3.

18. Single stranded nucleotide sequence being at least in part complementary to the messenger RNA transcribed from a gene as claimed in claims 1 to 3.

19. Single stranded nucleotide sequence as claimed in claim 18 which is antisense RNA.

20. Single stranded nucleotide sequence being at least in part complementary to the DNA or the cDNA from a gene as claimed in claims 1 to 3.

21. Single stranded nucleotide sequence as claimed in claims 18-20, further provided with a detectable label.

22. Nucleotide sequence as claimed in claims 18 to 21 for use as a probe in a method for detecting the occurrence of liver proliferation in a subject.

23. Nucleotide sequence as claimed in claim 22, **characterized in that** the method in which the nucleotide sequence is used as a probe comprises the steps of:
a) obtaining a sample of a tissue or body fluid; and
b) detecting the amount of messenger RNA transcribed from a gene as claimed in claims 1 to 3 in that sample in comparison to a reference sample by means of the probe.

24. Nucleotide sequence as claimed in claim 23, wherein the sample is a liver biopsy, plasma or serum.

25. Nucleotide sequence as claimed in claim 18, 20 or 21 for use as a probe for screening a liver cDNA or genomic library.
